(19) Europäisches Patentamt

European Patent Office

Office européen des brevets

(11) **EP 1 104 673 A1**

(12) **EUROPEAN PATENT APPLICATION**

(43) Date of publication:
**06.06.2001 Bulletin 2001/23**

(51) Int Cl.$^7$: **A61K 9/20**, A61K 31/495,
A61P 9/10

(21) Application number: **99402975.9**

(22) Date of filing: **30.11.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Bayer Classics**
**92806 Puteaux cedex (FR)**

(72) Inventors:
- **Joly, Stéphane Roger**
  **92200 Neuilly sur Seine (FR)**
- **Bouldoires, Vincent Pierre**
  **92200 Neuilly sur Seine (FR)**

(74) Representative: **Portal, Gérard et al**
**Cabinet Beau de Loménie**
**158, rue de l'Université**
**75340 Paris Cédex 07 (FR)**

(54) **A hydrogen carbonate-containing, desintegrating agent-free pharmaceutical composition**

(57) The present invention relates to novel galenic hydrogen carbonate-containing, disintegrating agent-free pharmaceutical compositions, and to their application in therapeutics. More particularly, the present invention notably relates to novel galenic hydrogen carbonate-containing, disintegrating agent-free pharmaceutical compositions notably having anti-ischaemic action, and to their application in therapeutics.

**Description**

FIELD OF THE INVENTION

The present invention relates to novel galenic hydrogen carbonate-containing, disintegrating agent-free pharmaceutical compositions, and to their application in therapeutics. More particularly, the present invention notably relates to novel galenic hydrogen carbonate-containing, disintegrating agent-free pharmaceutical compositions notably having anti-ischaemic action, and to their application in therapeutics.

BACKGROUND TO THE INVENTION

It is described in French patent No. 80 20919 that compositions based on trimetazidine hydrochloride in combination with a disintegrating agent (« a disintegrant»), which ensures a very rapid action would lead to a disappearance of the peripheral vasodilatory effect, that the blood flow rates or the tensional effects observed with low doses are found stabilised or practically stopped.
Moreover, for daily doses of the active principle of 20 to 80 mg, new effects appear which are adapted to the treatment of various illnesses.
The composition thus described is used for treating the metabolic effects of ischaemia.
Disintegrating agents such as modified corn starch and mannitol are cited amongst the excipients described in the French patent No. 80 20919 which can accelerate the action of the active principle by effecting a « flash effect ».

SUMMARY OF THE INVENTION

It has now been found in a surprising and entirely unexpected manner, and this constitutes an object of the present invention, that a composition containing an active pharmaceutical principle, *e.g.* based on trimetazidine dihydrochloride, without disintegrating agent, in combination with at least one diluting agent of the hydrogen carbonate type such as sodium bicarbonate, or potassium bicarbonate, has a disintegration time of less than 3 minutes according to the conditions described in the monograph of the European Pharmacopoeia 3$^{rd}$ Edition and USP 23 NF 18.
Thus, the present invention relates to a galenic, disintegrating agent-free, pharmaceutical composition of an active principle, notably of an active principle having anti-ischaemic action, characterised in that it comprises at least one diluting agent comprising a hydrogen carbonate. Said compositions can be administered in therapeutics in any form suitable for oral administration such as a tablet for example.
According to a first embodiment, the present invention relates to a galenic, disintegrating agent-free, pharmaceutical composition as mentioned above, characterised in that said hydrogen carbonate is in the form of a salt of a metal, such as sodium, potassium or calcium, for example.
According to a second embodiment, the present invention relates to a galenic, disintegrating agent-free, pharmaceutical composition as mentioned above, characterised in that it further comprises at least one flowing agent.
According to a third embodiment, the present invention relates to a galenic, disintegrating agent-free, pharmaceutical composition as mentioned above, characterised in that it further comprises at least one lubricant.
According to a fourth embodiment, the present invention relates to a galenic, disintegrating agent-free, pharmaceutical composition as mentioned above, characterised in that it further comprises at least one binding agent.
Further, in accordance with a fifth embodiment, the present invention relates to a galenic, disintegrating agent-free, pharmaceutical composition as mentioned above, characterised in that it is coated with a coating.
According to an advantageous embodiment, the above-mentioned active principle is trimetazidine, or a salt of trimetazidine, such as a hydrochloride for example, preferably a dihydrochloride.
According to another advantageous embodiment, the above-mentioned diluting agent is a diluting agent having the ability to accelerate the time of dissolution of said galenic pharmaceutical composition.
According to a first, currently preferred embodiment, the present invention relates to a galenic, disintegrating agent-free, pharmaceutical composition as mentioned above, characterised in that said flowing agent is anhydrous colloidal silica.
According to a second, currently preferred embodiment, the present invention relates to a galenic, disintegrating agent-free, pharmaceutical composition as mentioned above, characterised in that said lubricant is magnesium stearate.
According to a third, currently preferred embodiment, the present invention relates to a galenic, disintegrating agent-free, pharmaceutical composition as mentioned above, characterised in that said binding agent is selected from the group consisting of: copovidone ; povidone ; and hypromellose.
According to a fourth, currently preferred embodiment, the present invention relates to a galenic, disintegrating agent-free, pharmaceutical composition as mentioned above, characterised in that said coating is a coating of the aqueous film type.

Optionally, in accordance with the invention, an additional excipient or vehicle can be used which is acceptable for use via the oral route and which is compatible with said hydrogen carbonate of the invention in addition to the hydrogen carbonate of the invention. Such additional excipients or vehicles are known to the person skilled in the art, and additional diluting agents, other than those described above, can be cited, such as :

- a phosphate of a metal, such as sodium, potassium, or calcium for example, *e.g.* tricalcium phosphate, magnesium phosphate ;

  - silica and magnesium mixed silicates ;
  - microcrystalline cellulose ; and
  - lactose ;

  and an additional lubricant, such as:

  - talc; and
  - an acid, such as stearic acid for example, or one of its salts, other than magnesium stearate.

Hence, in accordance with yet another embodiment of the aspect, the present invention relates to a galenic, disintegrating agent-free, pharmaceutical composition as mentioned above, characterised in that it comprises, in addition to the hydrogen carbonate, an additional excipient or vehicle selected from the group consisting of an additional diluting agent, such as a phosphate of a metal, such as sodium, potassium, or calcium for example; silica or a magnesium mixed silicate; microcrystalline cellulose; lactose; and an additional lubricant, such as: talc; and an acid, such as stearic acid for example, or one of its salts, other than magnesium stearate.

According to a currently preferred advantageous embodiment, the present invention relates to a galenic, disintegrating agent-free, pharmaceutical composition as mentioned above, characterised in that trimetazidine dihydrochloride is present in an amount of between 10 and 30% by weight, preferably 15 to 25% by weight, and more preferably of between 20 and 23% by weight with respect to the total weight of the composition.

According to a second currently preferred advantageous embodiment, the present invention relates to a galenic, disintegrating agent-free, pharmaceutical composition as mentioned above, characterised in that said diluting agent is present in an amount of between 15 and 80% by weight, preferably of between 38 and 80% by weight, and more preferably of between 55 and 80% by weight with respect to the total weight of the composition.

It has been found in an entirely surprising and unexpected way that such a hydrogen carbonate-containing galenic composition without disintegrating agent has an accelerated dissolution.

DESCRIPTION OF A PREFERRED EMBODIMENT OF THE INVENTION

The preparations according to the invention preferentially contain 20 to 40 mg of an addition salt of 1-(2,3,4-trimethoxybenzyl)piperazine, with an inert excipient or vehicle. More particularly, compounds containing 20 mg of active principle will be used.

The therapeutic composition to be administered *via* the oral route will preferentially be presented in the form of a coated tablet.

The active principle is combined in this preparation with a diluting agent from the family of hydrogen carbonate salts and more particularly sodium bicarbonate.

EXAMPLES

The following Examples 1 to 10 in accordance with the present invention are tablets. These Examples illustrate the invention in a non-limiting way.

The tablets of the present invention are also produced by moist granulation as known to the person skilled in the art.

The other starting materials which are included in the composition of the tablet are used for shaping the tablet.

Such preparations are composed of the active principle (*e.g* trimetazidine dihydrochloride), of one or more diluting agents which integrate at least one hydrogen carbonate salt, and of a lubricating agent such as magnesium stearate for example.

The following can be essentially added to this composition :

- a binding agent (*e,g.* : copovidone, povidone, hypromellose ...)

- an anti-adhering agent (*e,g.* : talc, stearic acid ...)
- a flowing agent *(e.g. :* anhydrous colloidal silica ...)
- a coating of the aqueous film type.

The method of preparing these tablets is preferentially a direct compression process, but can be substituted by a dry or moist granulation process.

The manufacture equipment used is:

■ (DGM) ROTOLAB® dryer granulator mixer;
■ ERWEKA FGS AR 401 granulator calibrator, equipped with a stainless steel grid of opening diameter of mesh 1.0 mm ;
■ TURBULA powder mixer; and
■ FROGERAIS MR6 tabletting machine; equipped with 6R5 compression format.

The manufacture scheme used is :

Preparation Scheme :

| ORDER OF OPERATIONS | | UNDERGOING CONTROL |
|---|---|---|
| | | 0 ← Material - Atmosphere |
| | ACTIVE PRINCIPLE - EXCIPIENTS MIXTURE | I ← Speed - Duration |
| | | ← Conformity |
| PREPARATION OF MOULDING LIQUID | | II ← Conformity |
| | MOULDING | III ← Speed - Duration |
| | GRANULATION | IV |
| | DRYING | V ← Recording Duration - Temperature |
| | | ← Conformity |
| | CALIBRATION | VI ← Effectiveness |
| | | ← Particle size |
| PREPARATION OF THE INTERNAL PHASE | | VII ← Conformity |
| | MIXING | VIII ← Duration - Speed |
| | COMPRESSION | IX ← Conformity |
| PREPARATION OF THE COATING SOLUTION | | X ← Conformity |
| | COATING | XI ← Duration, temperature |
| | | ← Flow rates |
| | COATED TABLETS | XII ← Conformity |
| | | ← Appearence average mass |
| | | ← Disintegration |

The following Table I gives content, in mg, of each of the components of tablet Examples 1 to 10 and C1.

TABLE I

| Example | Active principle | NaHCO$_3$ 13/27 of the invention | NaHCO$_3$ 13/50 of the invention | Vivapur® PH102 | Aérosil® 200 | Plant magnesium stearate | sepifilm® 5341 red* | PEG 4000** | Theoretical mass Cps |
|---|---|---|---|---|---|---|---|---|---|
| 1 | 20.00 | 68.55 | - | - | 0.45 | 1.00 | - | - | 90.00 |
| 2 | 20.00 | - | 68.55 | - | 0.45 | 1.00 | - | - | 90.00 |
| 3 | 20.00 | - | 55.00 | 13.55 | 0.45 | 1.00 | - | - | 90.00 |
| 4 | 20.00 | - | 35.00 | 35.55 | 0.45 | 1.00 | - | - | 90.00 |
| 5 | 20.00 | - | 13.55 | 55.00 | 0.45 | 1.00 | - | - | 90.00 |
| 6a*** | 20.00 | - | 55.36 | 13.55 | 0.09 | 1.00 | - | - | 90.00 |
| 6b | 20.00 | - | 55.36 | 14.10 | 0.09 | 1.00 | - | - | 90.00 |
| 7a | 20.00 | - | 55.36 | 14.10 | 0.09 | 0.45 | - | - | 90.00 |
| 7b | 20.00 | - | 55.36 | 14.10 | 0.09 | 0.45 | - | - | 90.00 |
| 7c | 20.00 | - | 55.36 | 14.10 | 0.09 | 0.45 | - | - | 90.00 |
| 8a | 20.00 | - | 55.36 | 14.10 | 0.09 | 0.45 | 5.00 | - | 95.00 |
| 8b | 20.00 | - | 55.36 | 14.10 | 0.09 | 0.45 | 5.00 | - | 95.00 |
| 9 (shiny) | 20.00 | - | 55.36 | 14.10 | 0.09 | 0.45 | 5.00 | 1.00 | 96.00 |
| 10 | 20.00 | - | 55.36 | 13.88 | 0.09 | 0.67 | - | - | 90.00 |
| C1a | 20.00 | - | - | - | - | - | - | - | - |
| C1b | 20.00 | - | - | - | - | - | - | - | - |
| C1c | 20.00 | - | - | - | - | - | - | - | - |

* ( in 15% solution w/w)

** ( in 50% solution w/w)

*** a, b or c signify batch numbers
     C1 = Comparative example 1 (Example 1 of FR 2 490 963).

EP 1 104 673 A1

The following Table II gives characteristics of the final mixture of tablet Examples 1 to 10 and C1 :

TABLE II

| Example | Running (s) | Aptitude to packing (ml) | Volume mass | | centringparticle size distribution ($\mu$m) |
|---|---|---|---|---|---|
| | | | before packing | after packing | |
| 1 | 4.36 | 2 | 0.96 | 1 | 180 |
| 2 | 4.13 | 8 | 0.89 | 1 | 180 |
| 3 | infinite | 16 | 0.71 | 0.85 | 180 |
| 4 | 14.48 | 36 | 0.54 | 0.71 | 180 |
| 5 | 11.25 | 48 | 0.44 | 0.59 | 180 |
| 6a*** | 4.23 | 9 | 0.77 | 0.96 | 180 |
| 6b | 5.50 | 13 | 0.74 | 0.93 | 180 |
| 7a | 5.17 | 14 | 0.74 | 0.9 | 180 |
| 7b | 5.17 | 19 | 0.72 | 0.91 | 180 |
| 7c | - | - | - | - | - |
| 8a | - | - | - | - | - |
| 8b | - | - | - | - | - |
| 9 | - | - | - | - | - |
| 10 | 4.80 | 16 | 0.75 | 0.92 | 180 |
| C1a | - | - | - | - | - |
| C1b | - | - | - | - | - |
| C1c | - | - | - | - | - |

*** a, b or c signify batch numbers
C1 = Comparative example 1 (Example 1 of FR 2490 963).

The following Table III gives the physical characteristics of the finished tablet Examples 1 to 10 and C1 :

TABLE III

| Example | style | Average weight (mg) | Resistance to rupture | Deaggregation* | Friability | Observations |
|---|---|---|---|---|---|---|
| | | | (N) | (S) | (%) | |
| 1 | 6RS | 93.47 | not measurable | 50 | cleavage | |
| 2 | 6R5 | 89.34 | not measurable | 58 | cleavage | |
| 3 | 6R5 | 90.55 | 39.5 | 84 | 0.32 | |
| 4 | 6R5 | 90.57 | 81.1 | 225 | 0.15 | |
| 5 R1 | 6R5 | 91.72 | 134 | 602 | 0.02 | |
| 5 R2 | 6R5 | 89.72 | 100.1 | 421 | 0.01 | |
| 6a*** | 6R5 | 89.84 | 28 | 80 | 0.3 | demixing in container |
| 6b | 6R5 | 91.89 | 28 | 98 | 0.44 | |

* 0.1 N HCl, 37°C
*** a, b or c signify batch numbers
C1 = Comparative example 1 (Example 1 of FR 2 490 963).

TABLE III   (continued)

| Example | style | Average weight (mg) | Resistance to rupture | Deaggregation* | Friability | Observations |
|---|---|---|---|---|---|---|
| | | | (N) | (S) | (%) | |
| 7a | 6R5 | 93.6 | 30.7 | 73 | 0.36 | |
| 7b | 6R5 | 91.68 | 42.7 | 97 | 0.38 | |
| 7c | 6R5 | 91.27 | 36.13 | 82 | 0.61 | |
| 8a** | | 94.03 | 47.1 | 86 | | |
| 8** | | 91.88 | 56.9 | 150 | | |
| 9+ | | 92.91 | - | - | | |
| 10 | 6R5 | 92.68 | 27.6 | 85 | 0.34 | cleavage on compression |
| C1a | | 93.74 | 73.6 | 310 | | |
| C1b | | 94.55 | 63.9 | 293 | | |
| C1c | | 94.39 | 73.5 | 302 | | |

* 0.1 N HCl, 37°C

** non-coated tablets

+ coated tablets

More specifically, Comparative Examples C1a, C1b and C1c, (C1 batches a, b and c) are of the composition according to Example 1 of French Patent No. 80 20919, *viz* :

| COMPONENT | FUNCTION | AMOUNT in mg/unit |
|---|---|---|
| trimetazidine dihydrochloride | active principle | 20.00 |
| corn starch | | 26.00 |
| mannitol | | 34.00 |
| polyvidone | excipient | 4.00 |
| magnesium stearate | lubricant | 1.00 |
| talc | | 5.00 |
| coating film | | 5.00 |

EXAMPLE 11 : *IN VITRO* DISSOLUTION TEST of TABLET EXAMPLES 1 to 10 of the INVENTION.

The entirety of the pharmaceutical controls are carried out in accordance with the monographies of the European Pharmacopoeia 3rd Edition.

Method:

| | |
|---|---|
| Product | coated tablets having 20 mg of trimetazidine 2HCl |
| Analysis | trimetazidine 2HCl |
| Apparatus | PERKIN ELMER Lambda 20 UV/visible spectrophotometer |
| Dissolution medium | 0.1N HCl |
| Detection | 232.7 nm |
| Optical path | 10 mm |
| Number of tablets/cell | 1 |

(continued)

| Dissolution apparatus | SOTAX AT7 ON LINE |
|---|---|

Method of determination of trimetazidine dihydrochloride

Control solution:

100 mg of trimetazidine 2HCl, weighed out exactly, were dissolved in the dissolution medium, in a 500 ml graduated flask (class A), and was completed to thr graduation line with the same solvent. 5 ml of the resulting solution were taken and introduced into a 50 ml graduated flask class A) and was completed to the graduation line with the dissolution medium.
The diluted solution is filtered on a nylon/polypropylene syringe filter of 0.45 μm porosity.

Technique :

The absorbances obtained for each of the solutions was measured in referring to the F105 functioning method.

Calculation:

i.e.:

$A_1$ : the value of absorbance of trimetazidine 2HCl obtained for the control solution,
$A_2$: the value of absorbance of trimetazidine 2HCl obtained for the test solution,
$ts_1$ : test sample of trimetazidine 2HCl in mg
$ts_2$ : test sample of product to be analysed in mg
$tw_1$ : theoretical weight of trimetazidine 2HCl in mg
$tw_2$ : average weight of the batch of product to be analysed in mg
T : the titre of trimetazidine 2HCl of the control solution in %

The trimetazidine 2HCl content in the finished product, expressed in milligrams per tablet (mg/tab) is given by the equation :

$$t = \frac{A_2}{A_1} \times \frac{tw_2}{ts_2} \times \frac{ts_1}{100} \times T$$

Results :

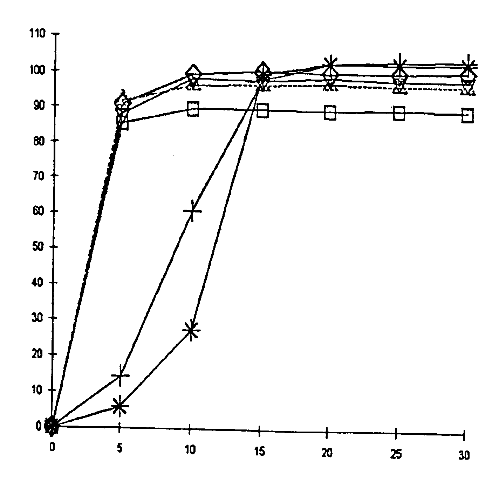

The hydrogen carbonate salts of the invention, as diluting agents, added to the active principle (*e.g* trimetazidine dihydrochloride) permit a rapid deaggregation of the tablets. In these various Examples, the method of preparation carried out is a direct compression. The finished product arising from these compositions has a deaggregation time of less than 1 minute.
Moreover, the dissolution profiles of these preparations enable at least 50% of the active principle to be released between ≈ 2.5 minutes and ≈ 12.5 minutes. (*cf*. graphs in Figures 1 to 3).
In all Figures, the experiment has been carried out with the tablet Example and 0.1 N HCl in a ratio of 60/1.

BRIEF EXPLANATION OF FIGURES

FIGURE 1: is a dissolution profile showing the results of an *in vitro* comparative dissolution test as described above, of tablet Examples of the invention. The average % of active principle (trimetazidine hydrochloride) released from the tablets is plotted up the ordinate, against time (in minutes) along the abscissa.
The curve bearing the marks + refers to tablet Example 3 ; the curve bearing stars refers to tablet Example 4 ; the curve bearing squares refers to tablet Example 6a ; the curve bearing △ marks refers to tablet Example 6b ; the curve bearing ▽ marks refers to tablet Example 7b ; and the curve bearing ◊ marks refers to a tablet Example 7a.

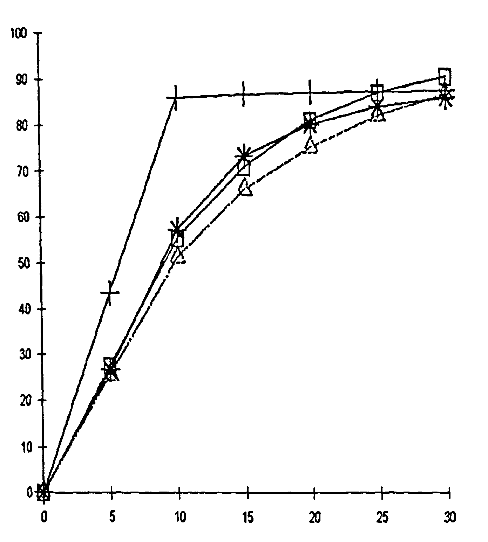

FIGURE 2 : is a dissolution profile showing the results of an *in vitro* comparative dissolution test as described above, of tablet Example 8b of the invention, and vastarel (20 mg). The average % of active principle (trimetazidine hydrochloride) released from the tablets is plotted up the ordinate, against time (in minutes) along the abscissa.

The curve bearing the marks + refers to tablet Example 8b ; the curve bearing stars refers to tablet Comparative Example C1a ; the curve bearing squares refers to tablet Comparative Example C1b ; the curve bearing Δ marks refers to tablet Comparative Example C1c.

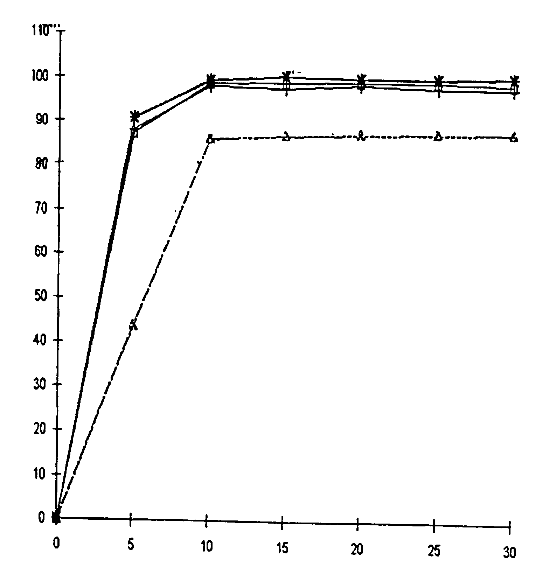

FIGURE 3 : is a dissolution profile showing the results of an in *vitro* comparative dissolution test as described above, of tablet Examples of the invention. The average % of active principle (trimetazidine hydrochloride) released from the tablets is plotted up the ordinate, against time (in minutes) along the abscissa.

The curve bearing the marks + refers to tablet Example 7b ; the curve bearing stars refers to a tablet Example 7a ; the curve bearing squares refers to tablet Example 8a ; and the curve bearing Δ marks refers to tablet Example 8b.

**Claims**

1. A galenic, disintegrating agent-free, pharmaceutical composition of an active principle, notably of an active principle having anti-ischaemic action, characterised in that it comprises at least one diluting agent comprising a hydrogen carbonate.

2. The galenic, disintegrating agent-free, pharmaceutical composition according to claim 1, characterised in that said hydrogen carbonate is in the form of a salt of a metal, such as sodium, potassium or calcium, for example.

3. The galenic, disintegrating agent-free, pharmaceutical composition according to claim 1 or 2, characterised in that it further comprises :

   - at least one flowing agent.

4. The galenic, disintegrating agent-free, pharmaceutical composition according to any one of claims 1 to 3, characterised in that it further comprises :

   - at least one lubricant.

5. The galenic, disintegrating agent-free, pharmaceutical composition according to any one of claims 1 to 4, characterised in that it further comprises :

   - at least one binding agent.

6. The galenic, disintegrating agent-free, pharmaceutical composition according to any one of claims 1 to 5, characterised in that it is coated with a coating.

7. The galenic, disintegrating agent-free, pharmaceutical composition according to any one of claims 1 to 6, characterised in that said active principle is trimetazidine, or a salt of trimetazidine, such as a hydrochloride for example, preferably a dihydrochloride.

8. The galenic, disintegrating agent-free, pharmaceutical composition according to any one of claims 1 to 7, characterised in that said diluting agent is a diluting agent having the ability to accelerate the time of dissolution of said galenic pharmaceutical composition.

9. The galenic, disintegrating agent-free, pharmaceutical composition according to any one of claims 2 to 8, characterised in that said flowing agent is anhydrous colloidal silica.

10. The galenic, disintegrating agent-free, pharmaceutical composition according to any one of claims 3 to 9, characterised in that said lubricant is magnesium stearate.

11. The galenic, disintegrating agent-free, pharmaceutical composition according to any one of claims 4 to 10, characterised in that said binding agent is selected from the group consisting of: copovidone; povidone; and hypromellose.

12. The galenic, disintegrating agent-free, pharmaceutical composition according to any one of claims 6 to 11, characterised in that said coating is a coating of the aqueous film type.

**13.** The galenic, disintegrating agent-free, pharmaceutical composition according to any one of claims 1 to 12, characterised in that it comprises, in addition to the hydrogen carbonate, an additional excipient or vehicle which is acceptable for use *via* the oral route and which is compatible with said hydrogen carbonate, in particular an additional excipient or vehicle selected from the group consisting of an additional diluting agent, such as a phosphate of a metal, such as sodium, potassium, or calcium for example; silica or a magnesium mixed silicate; microcrystalline cellulose; lactose; and an additional lubricant, such as : talc; and an acid, such as stearic acid for example, or one of its salts, other than magnesium stearate.

**14.** The galenic, disintegrating agent-free, pharmaceutical composition according to any one of claims 7 to 13, characterised in that trimetazidine dihydrochloride is present in an amount of between 10 and 30% by weight, preferably 15 to 25% by weight, and more preferably of between 20 and 23% by weight with respect to the total weight of the composition.

**15.** The galenic, disintegrating agent-free, pharmaceutical composition according to any one of claims 1 to 14, characterised in that said diluting agent is present in an amount of between 15 and 80% by weight, preferably of between 38 and 80% by weight, and more preferably of between 55 and 80% by weight with respect to the total weight of the composition.

**16.** The galenic, disintegrating agent-free, pharmaceutical composition according to any one of claims 1 to 15, characterised in that it is formulated as a tablet.

European Patent
Office

**EUROPEAN SEARCH REPORT**

Application Number

EP 99 40 2975

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | GB 2 084 019 A (SCIENCE UNION ET CIE, SOCIÉTÉ FRANCAISE DE RECHERCHE MÉDICALE) 7 April 1982 (1982-04-07) * page 1, line 1 - page 3, line 24 * | 1-16 | A61K9/20 A61K31/495 A61P9/10 |
| Y | FR 2 666 508 A (GLAXO GROUP LIMITED) 13 March 1992 (1992-03-13) * page 2, line 20 - line 33 * * page 6; example 2 * | 1-16 | |
| A | WO 96 02237 A (VACHER) 1 February 1996 (1996-02-01) * page 6; example 2 * | 1-16 | |

|  |
|---|
| TECHNICAL FIELDS SEARCHED (Int.Cl.7) |
| A61K A61P |

The present search report has been drawn up for all claims

| Place of search | Date of completion of the search | Examiner |
|---|---|---|
| THE HAGUE | 12 May 2000 | Benz, K |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

## ANNEX TO THE EUROPEAN SEARCH REPORT
## ON EUROPEAN PATENT APPLICATION NO.

EP 99 40 2975

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2000

| Patent document cited in search report | | Publication date | Patent family member(s) | | Publication date |
|---|---|---|---|---|---|
| GB 2084019 | A | 07-04-1982 | FR | 2490963 A | 02-04-1982 |
| | | | AU | 550761 B | 10-04-1986 |
| | | | AU | 7574481 A | 08-04-1982 |
| | | | BE | 890568 A | 30-03-1982 |
| | | | CA | 1169775 A | 26-06-1984 |
| | | | CH | 650675 A | 15-08-1985 |
| | | | DE | 3139005 A | 06-05-1982 |
| | | | GR | 75803 A | 02-08-1984 |
| | | | IE | 51764 B | 18-03-1987 |
| | | | IT | 1171557 B | 10-06-1987 |
| | | | LU | 83654 A | 14-04-1982 |
| | | | NZ | 198435 A | 03-02-1984 |
| | | | US | 4407801 A | 04-10-1983 |
| | | | ZA | 8106766 A | 29-09-1982 |
| FR 2666508 | A | 13-03-1992 | AT | 405134 B | 25-05-1999 |
| | | | AT | 180191 A | 15-10-1998 |
| | | | AU | 640816 B | 02-09-1993 |
| | | | AU | 8375791 A | 19-03-1992 |
| | | | BE | 1005115 A | 27-04-1993 |
| | | | CA | 2050970 A | 12-03-1992 |
| | | | CH | 683068 A | 14-01-1994 |
| | | | CY | 1988 A | 05-09-1997 |
| | | | DE | 4130061 A | 12-03-1992 |
| | | | DK | 159191 A | 12-03-1992 |
| | | | WO | 9317679 A | 16-09-1993 |
| | | | EP | 0558779 A | 08-09-1993 |
| | | | GB | 2248185 A,B | 01-04-1992 |
| | | | HK | 60894 A | 08-07-1994 |
| | | | HU | 9500511 A | 30-10-1995 |
| | | | IE | 65049 B | 04-10-1995 |
| | | | IL | 99428 A | 18-06-1996 |
| | | | IT | 1249696 B | 09-03-1995 |
| | | | JP | 6092849 A | 05-04-1994 |
| | | | MX | 9101009 A | 04-05-1992 |
| | | | NL | 9101533 A | 01-04-1992 |
| | | | NZ | 239731 A | 26-10-1993 |
| | | | RO | 112084 A | 30-05-1997 |
| | | | SE | 509694 C | 22-02-1999 |
| | | | SE | 9102604 A | 12-03-1992 |
| | | | US | 5456925 A | 10-10-1995 |
| | | | ZA | 9107176 A | 24-06-1992 |
| WO 9602237 | A | 01-02-1996 | FR | 2722408 A | 19-01-1996 |
| | | | AU | 2984395 A | 16-02-1996 |
| | | | EP | 0725631 A | 14-08-1996 |

EPO FORM P0459

For more details about this annex : see Official Journal of the European Patent Office, No. 12/82